# EUROPEAN PATENT SPECIFICATION

(11) **EP 4 397 630 B1**
(45) Date of publication and mention of the grant of the patent: **26.11.2025**
(21) Application number: 23863414.1
(22) Date of filing: 01.09.2023
(51) Int. Cl.: C02F 1/58, C02F 1/04, B01D 3/32, C07C 29/14, C07C 29/74, C07C 31/20, C02F 101/16, C02F 1/26, B01D 3/14, C02F 11/16, C02F 9/00, C02F 103/36, C07C 29/141, C07C 29/80, C07C 45/75, C07C 45/80, C07C 45/82

(54) **METHOD FOR TREATING NEOPENTYL GLYCOL WASTEWATER**
VERFAHREN ZUR BEHANDLUNG VON NEOPENTYLGLYKOLABWASSER
PROCÉDÉ DE TRAITEMENT D'EAUX USÉES DE NÉOPENTYLEGLYCOL

(30) Priority: 08.09.2022 KR 20220114489; 25.08.2023 KR 20230112002
(43) Date of publication of application: 10.07.2024
(73) Proprietor: LG Chem, Ltd., Yeongdeungpo-gu Seoul 07336 (KR)
(72) Inventor: KIM, Sung Kyun, Daejeon 34122 (KR); LEE, Sung Kyu, Daejeon 34122 (KR); LEE, Min Wook, Daejeon 34122 (KR)
(74) Representative: Goddar, Heinz J.
(86) International application number: PCT/KR2023/013051
(87) International publication number: WO 2024/053938

(56) References cited:
- CN-A- 112 174 794
- JP-A- 2016 505 074
- JP-A- H0 782 192
- JP-A- H0 782 192
- JP-A- H09 110 792
- KR-A- 20170 029 311
- US-B2- 10 093 603

## Description

### [Technical Field]

### CROSS-REFERENCE TO RELATED APPLICATIONS

The present application claims the benefit of priorities to Korean Patent Application No. 10-2022-0114489, filed on September 8, 2022, and Korean Patent Application No. 10-2023-0112002, filed on August 25, 2023.

### Technical Field

The present invention relates to a method for treating wastewater of neopentyl glycol, and more particularly, to a method for treating wastewater produced during a neopentyl glycol preparation process.

### [Background Art]

Neopentyl glycol may be generally prepared by performing an aldol condensation reaction of isobutyl aldehyde and formaldehyde in the presence of a catalyst to form hydroxypivaldehyde and proceeding a hydrogenation reaction of the hydroxypivaldehyde.

However, since the formaldehyde is used in an aqueous solution state for securing reactivity and flowability, it includes a large amount of water. Therefore, water included in the formaldehyde aqueous solution is treated as wastewater after an aldol condensation reaction. A catalyst which is not separated in an aldol purification process is introduced to the wastewater, and since the catalyst includes an amine group, it causes an occurrence of nitrogen oxides (NOₓ).

That is, in treating the wastewater, an environmental pollution problem is caused, and as an added catalyst is introduced to the wastewater and discarded, production costs increase during a process of continuously adding a new catalyst.

Therefore, a process which is environmentally friendly and may further improve economic feasibility by recovering the catalyst from the discarded wastewater and reusing it should be introduced. US10093603B2 relates to an economical apparatus and method for preparing glycol which reduces loss of a raw material and provides a high neopentyl glycol yield while inhibiting generation of by-products.

### [Disclosure]

### [Technical Problem]

In order to solve the problems mentioned in the Background Art, an object of the present invention is to provide a method for treating wastewater of neopentyl glycol which allows neopentyl glycol to be more obtained, is environmentally friendly in the overall process, and may further improve economic feasibility.

### [Technical Solution]

In one general aspect, provided is a method for treating wastewater of neopentyl glycol includes: an aldol reaction process of performing an aldol condensation reaction between a formaldehyde aqueous solution and isobutyl aldehyde in the presence of a catalyst to obtain a first reaction product including hydroxypivaldehyde; an aldol extraction process of bringing the first reaction product into contact with an extractant to obtain a raffinate including a catalyst salt and an extract including hydroxypivaldehyde; an aldol purification process of distilling the extract to be separated into wastewater including the catalyst, unreacted isobutyl aldehyde and the catalyst, and hydroxypivaldehyde; a hydrogenation reaction process of hydrogenating the hydroxypivaldehyde separated in the aldol purification process to obtain a second reaction product including neopentyl glycol; and a neopentyl glycol purification process of distilling the second reaction product to be separated into the extractant and the catalyst, wastewater including the catalyst, and neopentyl glycol, and supplying the separated extractant and catalyst to an extractant recovery column in which an extractant recovery process is performed to separate the wastewater including the catalyst and obtain the separated neopentyl glycol, and includes: recovering the wastewater including the catalyst from one or more processes of the aldol purification process, the neopentyl glycol purification process, and the extractant recovery process and supplying the wastewater to a volatile organic compound separation column; distilling the wastewater including the catalyst in the volatile organic compound separation column to obtain an upper discharge stream including the catalyst and a lower discharge stream including wastewater from which the catalyst has been removed; and supplying the lower discharge stream from the volatile organic compound separation column to a wastewater treatment system to treat the wastewater.

### [Advantageous Effects]

According to the method for treating wastewater of neopentyl glycol of the present invention, wastewater including a catalyst is supplied to a volatile organic compound separation column and distilled, thereby separating the catalyst. Since production of nitrogen oxides (NOₓ) may be significantly decreased by supplying the wastewater from which the catalyst has been separated to a wastewater treatment system at a latter stage, environmental pollution may be reduced.

In addition, the catalyst separated in the volatile organic compound separation column may be circulated to an aldol purification process to reuse the catalyst as a raw material in the aldol reaction process. Thus, production costs for preparing neopentyl glycol may be reduced to improve economic feasibility of the entire process.

### [Description of Drawings]

FIG. 1 is a process diagram showing a method for treating wastewater produced during a neopentyl glycol preparation process according to an exemplary embodiment of the present invention.
FIG. 2 is a process flow diagram showing a method for preparing neopentyl glycol according to an exemplary embodiment of the present invention.
FIG. 3 is a process diagram showing a method for treating wastewater produced during a neopentyl glycol preparation process according to the comparative example of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in a process or may refer to a fluid itself flowing in a pipe. Specifically, the "stream" may refer to both a fluid itself flowing in a pipe connecting each device and a fluid flow. In addition, the fluid may refer to a gas or liquid, and a case in which a solid substance is included in the fluid is not excluded.

Meanwhile, in the devices such as a separation column, an extraction column, a purification column, a distillation column or a distillation tower, and a recovery column in the present invention, unless otherwise particularly stated, a "lower portion" of the device refers to a point at a height of 95% to 100% from top to bottom of the device, specifically a lowest part (bottom). Likewise, unless otherwise particularly stated, an "upper portion" of the device refers to, a point at a height of 0% to 5% from the top to the bottom of the device, specifically the highest part (top).

Meanwhile, in the devices such as a separation column, an extraction column, a purification column, a distillation column, and a recovery column in the present invention, unless otherwise particularly stated, an operating temperature of the device may refer to a lower temperature of the device. Likewise, unless otherwise particularly stated, an operating pressure of the device may refer to upper pressure of the device.

Hereinafter, the present invention will be described in more detail for better understanding of the present invention.

According to an exemplary embodiment of the present invention, provided is a method for treating wastewater of neopentyl glycol including: an aldol reaction process of performing an aldol condensation reaction between a formaldehyde aqueous solution and isobutyl aldehyde in the presence of a catalyst to obtain a first reaction product including hydroxypivaldehyde; an aldol extraction process of bringing the first reaction product into contact with an extractant to obtain a raffinate including a catalyst salt and an extract including hydroxypivaldehyde; an aldol purification process of distilling the extract to be separated into wastewater including the catalyst, unreacted isobutyl aldehyde and the catalyst, and hydroxypivaldehyde; a hydrogenation reaction process of hydrogenating the hydroxypivaldehyde separated in the aldol purification process to obtain a second reaction product including neopentyl glycol; and a neopentyl glycol purification process of distilling the second reaction product to be separated into the extractant and the catalyst, wastewater including the catalyst, and neopentyl glycol, and supplying the separated extractant and catalyst to an extractant recovery column in which an extractant recovery process is performed to separate the wastewater including the catalyst and obtain the separated neopentyl glycol, and including: recovering the wastewater including the catalyst from one or more processes of the aldol purification process, the neopentyl glycol purification process, and the extractant recovery process and supplying the wastewater to a volatile organic compound separation column; distilling the wastewater including the catalyst in the volatile organic compound separation column to obtain an upper discharge stream including the catalyst and a lower discharge stream including wastewater from which the catalyst has been removed; and supplying the lower discharge stream from the volatile organic compound separation column to a wastewater treatment system to treat the wastewater.

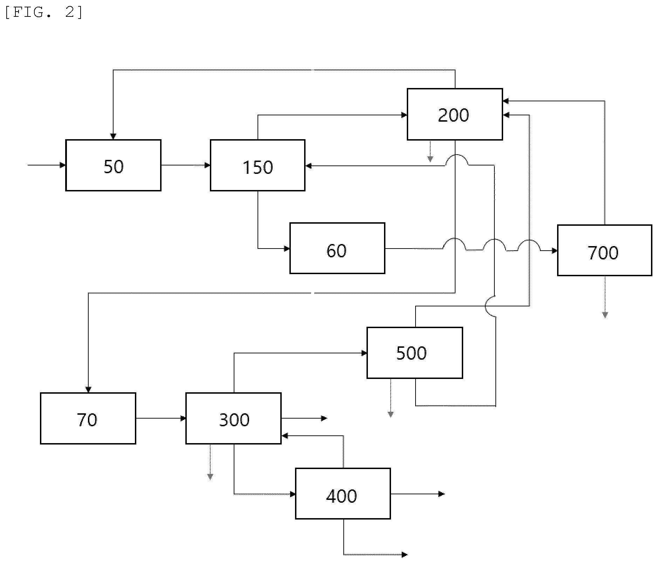

More specifically, the present invention will be described in detail referring to FIG. 2.

First, the method for treating wastewater of neopentyl glycol according to an exemplary embodiment of the present invention may include an aldol reaction process 50 of performing an aldol condensation reaction between a formaldehyde (FA) aqueous solution and isobutyl aldehyde (IBAL) in the presence of a catalyst to obtain a first reaction product including hydroxypivaldehyde (HPA).

Specifically, the aldol reaction process 50 may be performed in an aldol reactor in which the aldol condensation reaction is performed, and since the formaldehyde is used in an aqueous solution state for securing reactivity and flowability, it may include a large amount of water. The formaldehyde aqueous solution may include water in an amount of 40 to 64 wt%, more specifically 45 to 55 wt% with respect to the total weight of the formaldehyde aqueous solution. In addition, methanol for preventing polymerization of formaldehyde may be further included. In this case, the amount of methanol may be 0.1 to 15 wt%, more specifically 0.1 to 5 wt% with respect to the total weight of formaldehyde aqueous solution.

Herein, formalin may be used as the formaldehyde aqueous solution, and a concentration of formaldehyde of 35 to 45 wt% may be effective in terms of reducing wastewater.

In addition, the catalyst may be an amine-based compound. Specifically, a tertiary amine compound such as trialkylamine, trimethylamine, triethylamine, tripropylamine, triisopropylamine, and tributylamine may be appropriate. More specifically, the catalyst may include triethyl amine (TEA). In the present invention, since TEA is most efficient in the aldol condensation reaction, it may be used as the catalyst.

Specifically, in the aldol condensation reaction performed in the aldol reaction process 50, an aldol condensation reaction temperature may be 70°C to 100°C, and an aldol condensation reaction time may be 0.1 hours to 3 hours. In the aldol condensation reaction conditions as such, an aldol condensation reaction between a formaldehyde aqueous solution and IBAL may be performed in the presence of a catalyst to produce a catalyst salt and HPA.

Herein, the catalyst salt may be produced by reacting formic acid produced from a Cannizzaro side reaction occurring in the aldol condensation reaction and the catalyst.

In addition, hydroxypivalic acid-neopentyl glycol ester (HPNE) may be produced by a Tishchenko reaction which is another side reaction of the aldol condensation reaction.

Therefore, in the aldol reaction process 50, a first reaction product including the catalyst salt, HPNE, and HPA may be obtained, and the first reaction product may be supplied to an aldol extraction column in which an aldol extraction process 150 is performed.

The method for treating wastewater of neopentyl glycol according to an exemplary embodiment of the present invention may include an aldol extraction process 150 of bringing the first reaction product into contact with an extractant to obtain a raffinate including a catalyst salt and an extract including hydroxypivaldehyde.

Specifically, in the aldol extraction process 150, the first reaction product including the catalyst salt, HPNE, and HPA may be brought into contact with an extractant to obtain an organic extract including unreacted IBAL, an extractant, and HPA and a liquid raffinate including the catalyst salt. Herein, the unreacted IBAL may be IBAL which is not aldol condensation reacted in the aldol reaction process 50. The extract may be supplied to an aldol purification column in which an aldol purification process 200 is performed, and the raffinate may be supplied to a saponification reactor in which a saponification reaction process 60 is performed.

Herein, the extractant may be aliphatic alcohols, and preferably, may include 2-ethylhexanol (2-EH). Since HPA included in the first reaction product is soluble in the 2-EH, it may be preferably used in the aldol extraction process 150 using an extraction device according to a liquid-liquid contact method.

Meanwhile, an operating temperature of one or two or more aldol extraction columns in which the aldol extraction process 150 is performed may be 40°C to 90°C. By operating the aldol extraction column within the temperature range, it may be easy to separate the phase into an organic phase and a liquid phase.

The method for treating wastewater of neopentyl glycol according to an exemplary embodiment of the present invention may include a saponification reaction process 60 of saponifying the raffinate obtained in the aldol extraction process 150 to reduce the catalyst salt to the catalyst.

In the saponification reactor in which the saponification reaction process 60 is performed, the extract including the catalyst salt may be saponified to reduce the catalyst salt to the catalyst. The saponification reaction performed in the saponification reactor may be performed by a reaction of an inorganic strong base such as separately added sodium hydroxide (NaOH) and the catalyst salt, thereby efficiently reusing a reduced catalyst.

The reduced catalyst may be passed through a catalyst recovery process 700, and in the catalyst recovery process 700, the catalyst reduced in the catalyst recovery column may be distilled to separate the catalyst and wastewater, and the separated catalyst may be circulated to the aldol purification process 200.

Meanwhile, when the separated catalyst is supplied to an aldol reactor in which an aldol reaction process 50 is performed, a side reaction may occur excessively in the aldol reaction process 50 to produce a large amount of high-boiling point nitrogen compounds which are by-products, and these are introduced to a volatile organic compound (VOC) separation column described later to cause an increase in nitrogen oxides (NOₓ). Therefore, the separated catalyst is circulated to the aldol purification process 200 rather than directly supplied to the aldol reactor in which the aldol reaction process 50 is performed, thereby further decreasing the side reaction in the aldol reaction process 50 to reduce production of by-products, and thus, further minimizing an occurrence of NOₓ. Furthermore, the separated catalyst may be purified in the aldol purification column in which the aldol purification process 200 is performed, and may be reused as a higher-purity catalyst.

Meanwhile, the present invention may further include supplying wastewater separated from the catalyst recovery process 700 to a VOC separation column. Thus, when a part of the catalyst which is not separated in the catalyst recovery process 700 is included in the separated wastewater, the part of the catalyst included in the separated wastewater may be recovered in the VOC separation column.

The method for treating wastewater of neopentyl glycol according to an exemplary embodiment of the present invention may include the aldol purification process 200 of distilling the extract to separate wastewater including the catalyst, unreacted isobutyl aldehyde and the catalyst, and hydroxypivaldehyde.

Specifically, the aldol purification process 200 may be a process of distilling the catalyst recovered from the catalyst recovery process 700 and the extract separated from the aldol extraction process 150 to separate wastewater including the catalyst, unreacted IBAL and the catalyst, and HPA and the extractant. Herein, a distillate to be distilled in the aldol purification process 200 may further include the catalyst separated from an extractant recovery process 500 and an upper discharge stream from a volatile organic compound separation column described later, in addition to the catalyst recovered from the catalyst recovery process 700 and the extract separated from the aldol extraction process 150.

More specifically, the aldol purification process 200 may be performed by two or more columns. One or more of the two or more columns may include the aldol purification column and the remaining one or more may include a wastewater separation column.

First, when the aldol purification column is configured in one aldol purification column, the unreacted IBAL and the catalyst may be separated from an upper portion, and the HPA and the extractant may be separated from a lower portion of the one aldol purification column. Meanwhile, when the aldol purification column is configured in two or more aldol purification columns, it may comprise one or more aldol purification columns which separate unreacted IBAL to the upper portion and one or more aldol purification columns which separate the catalyst to the upper portion. A stream including HPA or the extractant may be separated and discharged into the lower portion of the two or more aldol purification columns. In addition, unreacted IBAL and/or the catalyst separated to the upper portion in the one or two or more aldol purification columns may be separated with water.

Meanwhile, the one or more wastewater separation columns may perform a function of separating wastewater (water) from the unreacted IBAL, the catalyst, HPA, and the extractant. Therefore, the stream supplied to the wastewater separation column may be a stream including water separated in one or more aldol purification columns. Furthermore, the wastewater recovered from the wastewater separation column may be supplied to a wastewater tank 10 described later. Herein, the wastewater supplied to the wastewater tank 10 may include a small amount of the catalyst which is not separated in the wastewater separation column and unreacted IBAL.

According to the present invention, in the aldol purification process 200, unreacted IBAL and the catalyst separated in the aldol purification process 200 may be circulated to the aldol reaction process 50. Thus, since the catalyst and IBAL used in the aldol reaction process 50 are recycled, the amount of a newly added raw material may be reduced and production costs used during the process may be reduced.

The method for treating wastewater of neopentyl glycol according to an exemplary embodiment of the present invention may include a hydrogenation reaction process 70 of hydrogenating the hydroxypivaldehyde separated in the aldol purification process 200 to obtain a second reaction product including neopentyl glycol.

In the hydrogenation reaction process 70, a hydrogenation reaction of hydrogen which is added separately from HPA separated in the aldol purification process 200 proceeds, thereby obtaining the second reaction product including NPG. Herein, the hydrogenation reaction may proceed at a hydrogen pressure of 689.48 to 10342.14 kPa(100 to 1500 psig, pounds per square inch gauge pressure) and a reaction temperature of 100°C to 200°C.

In addition, the hydrogenation reaction may be performed in the presence of a hydrogenation reaction catalyst. A nickel catalyst or a copper-based catalyst may be used as the hydrogenation reaction catalyst. The nickel catalyst may be present an amount of 2 to 10 wt% with respect to the weight of HPA. An example of the copper-based catalyst may be a CuO/BaO/SiO catalyst, and the CuO/BaO/SiO catalyst may be a catalyst of (CuO)ₓ(BaO)_{y}(SiO)_{z} (x, y, and z are wt%, and x:y:z = 10-50:0-10:40-90, 10-50:1-10:40-89, or 29-50:1-10:40-70). The sum of x and y is preferably 20 to 50 (wt%) or 30 to 50 (wt%), based on the sum of x, y, and z (100 wt%), and in this range, performance of the hydrogenation reaction catalyst is excellent and an effect of long life is shown.

When the hydrogenation reaction of hydrogen and HPA proceeds as such, NPG may be produced. Therefore, the second reaction product including the catalyst, the extractant, the NPG, and the HPNE may be obtained in the hydrogenation reaction process 70. Herein, the HPNE may be produced as a by-product of an aldol condensation reaction in the aldol reaction process 50. Subsequently, a desired product, neopentyl glycol, may be obtained by the NPG purification process 300 of the second reaction product.

The method for treating wastewater of neopentyl glycol according to an exemplary embodiment of the present invention may include neopentyl glycol purification process 300: distilling the second reaction product to be separated into the extractant and the catalyst, wastewater including the catalyst, and neopentyl glycol, and the separated extractant and the catalyst are supplied to an extractant recovery column in which an extractant recovery process 500 is performed to separate the wastewater including the catalyst and the separated neopentyl glycol is obtained.

Specifically, the NPG purification process 300 may be a process of distilling the second reaction product to be separated into wastewater including the catalyst, the extractant and the catalyst, and NPG. The separated extractant and catalyst may be introduced to an extractant recovery process 500 described later, and the NPG may be obtained in the NPG purification process 300 as an intended product.

In addition to this, in the NPG purification process 300, HPNE may be further separated from the second reaction product, and a stream including HPNE may be introduced to a hydroxypivalic acid-neopentyl glycol ester (HPNE) purification process.

Meanwhile, the NPG purification process 300 may be performed by two or more columns as in the aldol purification process 200. One or more of the two or more columns may include the NPG purification column and the remaining one or more columns may include a wastewater separation column.

First, when the NPG purification column is configured in one NPG purification column, the extractant and the catalyst may be separated from an upper portion, HPNE may be separated from a lower portion, and NPG may be separated from a side portion of the one NPG purification column, respectively. Meanwhile, when the NPG purification column is configured in two or more NPG purification columns, it may comprise one or more NPG purification columns which separate the extractant to the upper portion and one or more NPG purification columns which separate the catalyst to the upper portion. A stream including HPNE or NPG may be separated and discharged into the lower portion of two or more NPG purification columns. In addition, a stream including water, that is, a wastewater stream, may be separated in one or two or more NPG purification columns, separately with the catalyst, the extractant, HPNE, and NPG.

Meanwhile, the one or more wastewater separation columns may perform a function of separating the wastewater from the catalyst, the extractant, HPNE, and NPG. Therefore, the stream supplied to the wastewater separation column may be a wastewater stream separated from one or more NPG purification columns. Furthermore, the wastewater separation column may supply the wastewater separated as such to a wastewater tank 10 described later. Herein, the wastewater may include the catalyst which is not separated in the NPG purification column.

Meanwhile, the extractant recovery process 500 of the present invention may be a process of supplying the catalyst and the extractant separated from the neopentyl glycol purification process 300 to the catalyst recovery column, separating the wastewater including the catalyst, the catalyst, and the extractant, introducing the catalyst to the aldol purification process 200, and introducing the extractant to the aldol extraction process 150. Herein, the catalyst may be a small amount of the catalyst which is not separated in the aldol extraction process 150 and the aldol purification process 200 described above. Meanwhile, the extractant may be circulated to the aldol extraction process 150, thereby recovering the extractant, for example, 2-EH and reusing it in the aldol extraction process 150.

More specifically, the extractant recovery process 500 may be performed by a column, that is, an extractant recovery column. The catalyst may be separated from the upper portion and the extractant may be separated from the lower portion of the extractant recovery column, respectively. In addition, a stream including water, that is, a wastewater stream may be separated in the extractant recovery column, separately from the catalyst and the extractant. Furthermore, the wastewater stream separated in the extractant recovery column may be supplied to a wastewater tank 10 described later. Herein, the wastewater may include a small amount of the catalyst.

Meanwhile, in the HPNE purification process 400 according to an exemplary embodiment of the present invention, a stream including HPNE separated from the NPG purification process 300 may be distilled to separate a small amount of NPG and HPNE. Subsequently, a small amount of separated NPG may be refluxed to the NPG purification process 300, and the HPNE may be separately obtained.

Herein, the small amount of NPG is not recovered as a product in the NPG purification process and separated with HPNE, and since NPG may be further recovered from the HPNE purification process 400, the recovery rate of NPG may be further increased. In addition, the separately obtained HPNE may be used in various ways, and for example, may be used as a main raw material of coating and synthesizing polyester as a high value-added product. Since the HPNE may be used as a raw material in other processes, economic feasibility may be improved in terms of raw material use by the HPNE purification process 400.

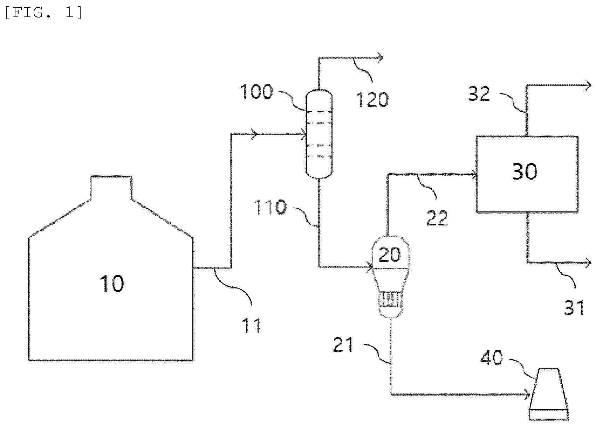

Hereinafter, the present invention will be described in more detail with reference to FIG. 1 according to an exemplary embodiment.

The method for treating wastewater of NPG according to an exemplary embodiment of the present invention may include: recovering wastewater including a catalyst from one or more of the aldol purification process, the neopentyl glycol purification process, and the extractant recovery process and supplying the wastewater to a volatile organic compound (VOC) separation column; obtaining an upper fraction including the catalyst and a lower fraction including the wastewater from which the catalyst has been removed in the VOC separation column; and supplying the lower fraction to a wastewater treatment system to treat the wastewater.

First, the method for treating wastewater of NPG according to an exemplary may include recovering wastewater including a catalyst from one or more processes of the aldol purification process, the neopentyl glycol purification process, and the extractant recovery process and supplying the wastewater to a VOC separation column 100.

Specifically, the wastewater separated in one or more columns performed in one or more processes of the aldol purification process, the NPG purification process, and the extractant recovery process may be introduced to a wastewater tank 10. In addition to this, the wastewater separated from the catalyst recovery process may also be introduced to the wastewater tank 10.

As such, the wastewater separated in a plurality of processes may be supplied to the wastewater tank 10 and supplied to the VOC separation column 100 as a discharge stream 11 from the wastewater tank. Herein, the wastewater may be derived from water included in the formaldehyde aqueous solution used in the aldol reaction process 50. In addition to this, the wastewater may include the catalyst and unreacted IBAL which are not separated in the aldol purification process.

Meanwhile, as described above, the catalyst in the present invention may be, for example, TEA, and since TEA includes an amine group, it may cause occurrence of nitrogen oxides (NOₓ). Since the NOₓ may be harmful in an environmental aspect, the TEA needs to be separated to decrease production of NOₓ. Therefore, in the present invention, separation of the catalyst may be performed through the VOC separation column 100, and the catalyst separated from the VOC separation column 100 may be recovered and reused.

The method for treating wastewater of neopentyl glycol according to an exemplary embodiment of the present invention may include distilling the wastewater including the catalyst in the VOC separation column to obtain an upper discharge stream including the catalyst and a lower discharge stream including the wastewater from which the catalyst has been removed.

In the VOC separation column 100, the discharge stream 11 from the wastewater tank may be distilled to be separated into an upper fraction including the catalyst and a lower fraction including the wastewater from which the catalyst has been removed. Thus, as described above, TEA which is the catalyst of the present invention to promote production of NOₓ is separated, thereby adjusting an amine component included in the lower fraction of the VOC separation column to decrease production of NOₓ.

Meanwhile, an operating temperature of the VOC separation column 100 may be 50°C or higher, 60°C or higher, or 70°C or higher and 160°C or lower, 180°C or lower, or 200°C or lower. In addition, an operating pressure of the VOC separation column 100 may be 66.69kPa(0.68 kg/cm²)or more, 147.1 kPa(1.50 kg/cm²) or more, or 196.13 kPa(2.00 kg/cm²) or more and 392.27 kPa(4.00 kg/ cm²) or less, 441.30 kPa(4.50 kg/cm²) or less, or 490.33 kPa(5.00 kg/cm²) or less.

The upper fraction of the VOC separation column 100 may be circulated to the aldol purification process through the upper discharge stream 120 from the VOC separation column. The upper fraction of the VOC separation column 100 includes the catalyst and unreacted IBAL, and the catalyst and the unreacted IBAL may be reused by circulating them to the aldol purification process. The catalyst may be recovered and reused as such, thereby decreasing the production of NOₓ to secure cost competitiveness of the process while being environmentally friendly.

Meanwhile, the lower fraction of the VOC separation column 100 including the wastewater from which the catalyst has been separated may be supplied to an evaporator 20 through the lower discharge stream 110 of the VOC separation column.

According to an exemplary embodiment of the present invention, an amount of the catalyst in the lower discharge stream 110 of the VOC separation column may be 5.0 wt% or less, more specifically 4.0 wt% or less or 3.0 wt% or less. The catalyst is included within the range, thereby decreasing the amine component in the stream introduced to a wastewater treatment system 30 described later to decrease production of NOₓ in the wastewater treatment system 30.

According to an exemplary embodiment of the present invention, the lower discharge stream from the VOC separation column is supplied to the evaporator 20, sludge is removed, and the upper fraction of the evaporator from which the sludge has been removed may be supplied to the wastewater treatment system 30.

The lower discharge stream 110 from the VOC separation column including the wastewater separated from the catalyst may be evaporated in the evaporator 20, thereby being separated into a lower fraction of the evaporator including the sludge and an upper fraction of the evaporator including gaseous components from which the sludge has been removed. The lower fraction of the evaporator may be supplied to a sludge dryer 40 through a lower discharge stream 21 from the evaporator, and the upper fraction of the evaporator may be supplied to the wastewater treatment system 30 through an upper discharge stream 22 from the evaporator.

Meanwhile, in the sludge dryer 40, the sludge included in the lower discharge stream 21 from the evaporator may be dried, and treated as wastes or burned to be used as an energy source of the process.

The method for treating wastewater of neopentyl glycol according to an exemplary embodiment of the present invention may include supplying the lower discharge stream from the VOC separation column to the wastewater treatment system and treating the wastewater.

Specifically, in the wastewater treatment system 30, the lower discharge stream from the VOC separation column which has passed through the evaporator 20, that is, the upper discharge stream 22 from the evaporator is wastewater-treated, thereby obtaining a waste gas discharge stream 32 including waste gas and a wastewater discharge stream 31 including the wastewater. Herein, the waste gas may be a gas from which an amine group has been removed.

More specifically, the wastewater treatment system is a system which performs a process for treating organic matter present in water, and a regeneration thermal oxidizer (RTO), a DeNOₓ system, and the like may be used for performing the wastewater treatment system.

The regeneration thermal oxidizer is a facility which may significantly reduce the operation costs of an incinerator by recovering heat produced during incineration through a ceramic filler which has a large surface area and may be used semi-permanently, after incinerating gaseous components containing organic matter. In addition, treatment efficiency of the regeneration thermal oxidizer is 99% or more which is very high and has a small secondary pollution factor.

Meanwhile, the DeNOₓ system is a system which removes nitrogen oxides by selective catalytic reduction (SCR), and the selective catalytic reduction (SCR) may pass the gaseous components including nitrogen oxides produced during the process through a catalyst layer with a reducing agent (ammonia or urea) to decompose the gaseous components into nitrogen and water vapor and discharge them to the atmosphere.

As described above, the catalyst, for example, TEA, is removed in advance through the VOC recovery column to remove TEA introduced to the wastewater treatment system, thereby decreasing the amount of NOₓ produced in the wastewater treatment system to environmentally friendly prepare NPG. In addition, the catalyst which is separated and recovered in the VOC recovery column is circulated to the aldol purification process and purified, whereby the purified catalyst may be in an appropriate state of being reused in the aldol reaction process 50.

As another example, the VOC separation column may be a dividing wall column (DWC). When the dividing wall column (DWC) is used as the VOC separation column, the evaporator and the wastewater treatment system at the latter stage is not needed separately, and thus, process may be simplified and may be managed efficiently. In addition, energy consumption by thermal integration may be reduced.

More specifically, the dividing wall column is in the form of being provided with a dividing wall in the distillation column, and since the space provided by the dividing wall is divided into two, functions such as a function of integrating two distillation columns into one may be performed. Therefore, the catalyst and unreacted IBAL may be separated as the upper fraction, the sludge may be separated as the lower fraction, and the wastewater may be separated as the side fraction.

The lower fraction of the VOC separation column including the sludge may be supplied to a sludge dryer through a lower discharge stream from the VOC separation column and dried, and the side fraction of the VOC separation column 100 including the wastewater may be discharged through the side discharge stream 130 from the VOC separation column.

In addition, the upper fraction of the VOC separation column including the catalyst and unreacted IBAL may be circulated to the aldol purification process as the upper discharge stream from the VOC separation column, as described above. The catalyst may be purified and recovered in the aldol purification process, and the recovered catalyst may be circulated to the aldol reaction process 50 and reused as a raw material of the aldol condensation reaction.

Hereinafter, the present invention will be described in more detail by the examples. However, the following examples are provided for illustrating the present invention. It is apparent to a person skilled in the art that various modifications and alterations may be made without departing from the scope and spirit of the present invention, and the scope of the present invention is not limited thereto.

### Examples

### Example 1

According to the process diagram illustrated in FIG. 1, a wastewater treatment process of neopentyl glycol (NPG) was simulated, using an Aspen Plus simulator available from Aspen.

In an aldol reactor, an aldol condensation reaction between a formaldehyde aqueous solution and isobutyl aldehyde was performed in the presence of a catalyst (triethylamine, TEA) to obtain a first reaction product including hydroxypivaldehyde (aldol reaction process).

The first reaction product was brought into contact with an extractant (2-ethylhexnaol, 2-EH) to obtain a raffinate including a catalyst salt, an extract including hydroxypivaldehyde, and wastewater including the catalyst (aldol extraction process).

The raffinate was saponified to reduce the catalyst salt to the catalyst, thereby obtaining a reduced catalyst (saponification reaction process). The reduced catalyst was distilled in a catalyst recovery column to separate wastewater including the catalyst and the catalyst, and the catalyst was recovered (catalyst recovery process).

The catalyst separated from the catalyst recovery process and the extract obtained from the aldol extraction process were distilled in the aldol purification column in which the aldol purification process was performed, thereby separating unreacted isobutyl aldehyde and the catalyst, wastewater including the catalyst, and hydroxypivaldehyde. The separated unreacted isobutyl aldehyde and the catalyst were circulated to the aldol reaction process (aldol purification process).

Meanwhile, the hydroxypivaldehyde separated from the aldol purification process was hydrogenated, thereby obtaining a second reaction product including neopentyl glycol (hydrogenation reaction process).

The second reaction product was distilled to separate wastewater including the catalyst, the extractant and the catalyst, neopentyl glycol, and hydroxypivalic acid-neopentyl glycol ester (HPNE), and the neopentyl glycol was obtained as a product (NPG purification process).

The extractant and the catalyst separated from the NPG purification process were passed through an extractant recovery process to separate wastewater including the catalysts, the extractant, and the catalyst, respectively. The extractant separated from the extractant recovery process was circulated to the aldol extraction process, and the catalyst separated from the extractant recovery process was circulated to the aldol purification process.

Meanwhile, a stream including HPNE separated from the NPG purification process was circulated to the HPNE purification process to be separated into NPG and HPNE. The NPG separated from the HPNE purification process was circulated to the NPG purification process, and the HPNE was separately obtained.

Wastewater including the catalyst was recovered from the catalyst recovery process, the aldol purification process, the neopentyl glycol purification process, and the extractant recovery process and introduced to a waste tank 10.

The wastewater including the catalyst introduced to the wastewater tank 10 was supplied to a volatile organic compound (VOC) separation column 100 as a discharge stream 11 from the wastewater tank. An upper fraction including the catalyst and a lower fraction including the wastewater from which the catalyst had been removed were separated in the VOC separation column 100. At this time, the operating temperature of the VOC separation column 100 was 110°C, and the operating pressure thereof was 107.87 kPa (1.1 kg/ cm²).

The upper fraction including the catalyst was circulated to the aldol purification process through the upper discharge stream 120 from the VOC separation column, and the lower fraction including the wastewater from which the catalyst had been removed was supplied to an evaporator 20 through the lower discharge stream 110 from the VOC separation column.

The lower discharge stream 110 from the VOC separation column was evaporated in the evaporator 20, thereby separating a lower fraction of the evaporator including sludge and an upper fraction of the evaporator including gaseous components from which the sludge had been separated. The lower fraction of the evaporator including the sludge was supplied to a sludge dryer 40 as the lower discharge stream 21 from the evaporator, and the upper fraction of the evaporator including gaseous components from which the sludge had been separated was supplied to the wastewater treatment system 30 as the upper discharge stream 22 from the evaporator.

In the wastewater treatment system 30, the upper discharge stream 22 from the evaporator was wastewater treated to separate waste gas and wastewater, the waste gas was discharged through the waste gas discharge stream 32, and the wastewater was discharged through the wastewater discharge stream 31.

### Example 2

In Example 2, wastewater of NPG was treated by the same process flow as in Example 1, except that the catalyst separated from the catalyst recovery process was circulated to the aldol reactor in which the aldol reaction process was performed, not the aldol purification process.

### Comparative Examples

### Comparative Example 1

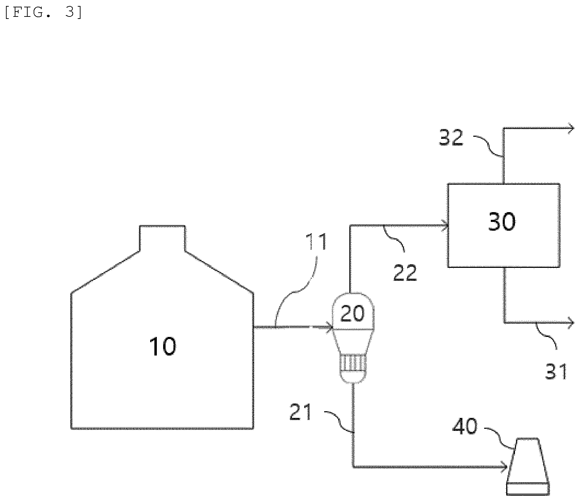

According to the process diagram illustrated in FIG. 3, a wastewater treatment process of neopentyl glycol (NPG) was simulated, using the Aspen Plus simulator available from Aspen.

In Comparative Example 1, NPG was prepared by the same process flow as in Example 1, except that the VOC separation column was not provided and the discharge stream 11 from the wastewater tank was supplied to the evaporator 20.

In the following Table 1, the TEA content in the feed stream of the examples and the comparative examples, and the TEA content and the amount of NOₓ produced in the wastewater treatment system are shown.

Specifically, the TEA content in the wastewater treatment system may refer to a ratio of the weight of TEA in the stream supplied to the wastewater treatment system to the total weight of the stream supplied to the wastewater treatment system (upper discharge stream from the evaporator).

Meanwhile, the amount of NOₓ produced represents a fraction of nitrogen oxides (NOₓ) included in the waste gas discharge stream 32 to the total weight of the waste gas discharge stream 32 of the wastewater treatment system.

**[Table 1]**

| | TEA content (wt%) in feed stream¹⁾ | TEA content (wt%) in wastewater treatment system | Amount of NOₓ produced (ppm) |
|---|---|---|---|
| Example 1 | 0.02 | 0 | 4 |
| Example 2 | 0.05 | 0.01 | 17 |
| Comparative Example 1 | 0.02 | 0.02 | 38 |
| ¹⁾ The feed stream was a discharge stream from the wastewater tank, and refers to a feed stream supplied to the VOC separation column in the examples and refers to a feed stream introduced to the evaporator in the comparative examples. | | | |

Referring to Table 1, it was confirmed that NOₓ production in the examples was decreased as compared with Comparative Example 1. However, it was confirmed that the VOC separation column was not provided in Comparative Example 1 and had highest NOₓ production as compared with the examples.

## Claims

1. A method for treating wastewater of neopentyl glycol, the method comprising:
an aldol reaction process of performing an aldol condensation reaction between a formaldehyde aqueous solution and isobutyl aldehyde in the presence of a catalyst to obtain a first reaction product including hydroxypivaldehyde;
an aldol extraction process of bringing the first reaction product into contact with an extractant to obtain a raffinate including a catalyst salt and an extract including hydroxypivaldehyde;
an aldol purification process of distilling the extract to be separated into wastewater including the catalyst, unreacted isobutyl aldehyde and the catalyst, and hydroxypivaldehyde;
a hydrogenation reaction process of hydrogenating the hydroxypivaldehyde separated in the aldol purification process to obtain a second reaction product including neopentyl glycol; and
a neopentyl glycol purification process of distilling the second reaction product to be separated into the extractant and the catalyst, wastewater including the catalyst, and neopentyl glycol, and supplying the separated extractant and catalyst to an extractant recovery column in which an extractant recovery process is performed to separate the wastewater including the catalyst and obtain the separated neopentyl glycol, and comprising:
recovering the wastewater including the catalyst from one or more processes of the aldol purification process, the neopentyl glycol purification process, and the extractant recovery process and supplying the wastewater including the catalyst to a volatile organic compound separation column;
distilling the wastewater including the catalyst in the volatile organic compound separation column to obtain an upper discharge stream including the catalyst and a lower discharge stream including wastewater from which the catalyst has been removed; and
supplying the lower discharge stream from the volatile organic compound separation column to a wastewater treatment system to treat the wastewater.

2. The method for treating wastewater of neopentyl glycol of claim 1, further comprising: circulating the upper discharge stream from the volatile organic compound separation column to the aldol purification process.

3. The method for treating wastewater of neopentyl glycol of claim 1, wherein the aldol purification process is a process of circulating the separated unreacted isobutyl aldehyde and the catalyst to the aldol reaction process.

4. The method for treating wastewater of neopentyl glycol of claim 1, further comprising a saponification reaction process of saponifying the raffinate obtained in the aldol extraction process to reduce the catalyst salt to the catalyst.

5. The method for treating wastewater of neopentyl glycol of claim 4, further comprising a catalyst recovery process of distilling the catalyst reduced in the saponification reaction process to separate wastewater and the catalyst and circulating the separated catalyst to the aldol purification process.

6. The method for treating wastewater of neopentyl glycol of claim 5, further comprising supplying the wastewater separated from the catalyst recovery process to the volatile organic compound separation column.

7. The method for treating wastewater of neopentyl glycol of claim 1, wherein the extractant recovery process is a process of supplying the catalyst and the extractant separated from the neopentyl glycol purification process to the catalyst recovery column, separating the wastewater including the catalyst, the catalyst, and the extractant, introducing the catalyst to the aldol purification process, and introducing the extractant to the aldol extraction process.

8. The method for treating wastewater of neopentyl glycol of claim 1,
wherein the neopentyl glycol purification process is a process of further separating hydroxypivalic acid-neopentyl glycol ester (HPNE) from the second reaction product, and
a hydroxypivalic acid-neopentyl glycol ester purification process of distilling a stream including hydroxypivalic acid-neopentyl glycol ester (HPNE) separated from the neopentyl glycol purification process and circulating a small amount of neopentyl glycol to the neopentyl glycol purification process to obtain hydroxypivalic acid-neopentyl glycol ester is further included.

9. The method for treating wastewater of neopentyl glycol of claim 1, wherein the catalyst includes triethyl amine (TEA).

10. The method for treating wastewater of neopentyl glycol of claim 1, wherein the extractant includes 2-ethylhexanol (2-EH).

11. The method for treating wastewater of neopentyl glycol of claim 1, wherein an amount of the catalyst in the lower discharge stream from the volatile organic compound separation column is 5.0 wt% or less.

12. The method for treating wastewater of neopentyl glycol of claim 1, further comprising supplying the lower discharge stream from the volatile organic compound separation column to an evaporator, removing sludge, and supplying an upper fraction of the evaporator from which the sludge has been removed to the wastewater treatment system.

## Patentansprüche

1. Verfahren zur Behandlung von Abwasser von Neopentylglykol, wobei das Verfahren umfasst:
einen Aldolreaktionsprozess des Durchführens einer Aldolkondensationsreaktion zwischen einer wässrigen Formaldehydlösung und Isobutylaldehyd in Gegenwart eines Katalysators, um ein erstes Reaktionsprodukt zu erhalten, das Hydroxypivaldehyd enthält;
einen Aldolextraktionsprozess des Inkontaktbringens des ersten Reaktionsprodukts mit einem Extraktionsmittel, um ein Raffinat, das ein Katalysatorsalz enthält, und einen Extrakt, der Hydroxypivaldehyd enthält, zu erhalten;
einen Aldolreinigungsprozess des Destillierens des zu trennenden Extrakts in Abwasser, das den Katalysator, nicht umgesetzten Isobutylaldehyd und den Katalysator und Hydroxypivaldehyd enthält;
einen Hydrierungsreaktionsprozess des Hydrierens des in dem Aldolreinigungsprozess getrennten Hydroxypivaldehyds, um ein zweites Reaktionsprodukt zu erhalten, das Neopentylglykol enthält; und
einen Neopentylglykolreinigungsprozess des Destillierens des zu trennenden zweiten Reaktionsprodukts in das Extraktionsmittel und den Katalysator, Abwasser, das den Katalysator enthält, und Neopentylglykol und des Zuführens des getrennten Extraktionsmittels und Katalysators zu einer Extraktionsmittelrückgewinnungskolonne, in der ein Extraktionsmittelrückgewinnungsprozess durchgeführt wird, um das Abwasser, das den Katalysator enthält, zu trennen und das getrennte Neopentylglykol zu erhalten, und umfassend:
Rückgewinnen des Abwassers, das den Katalysator enthält, aus einem oder mehreren Prozessen des Aldolreinigungsprozesses, des Neopentylglykolreinigungsprozesses und des Extraktionsmittelrückgewinnungsprozesses und Zuführen des Abwassers, das den Katalysator enthält, zu einer Trennkolonne für flüchtige organische Verbindungen;
Destillieren des Abwassers, das den Katalysator enthält, in der Trennkolonne für flüchtige organische Verbindungen, um einen oberen Austragsstrom, der den Katalysator enthält, und einen unteren Austragsstrom, der Abwasser enthält, aus dem der Katalysator entfernt wurde, zu erhalten; und
Zuführen des unteren Austragsstroms aus der Trennkolonne für flüchtige organische Verbindungen zu einem Abwasserbehandlungssystem, um das Abwasser zu behandeln.

2. Verfahren zur Behandlung von Abwasser von Neopentylglykol nach Anspruch 1, ferner umfassend: Zirkulieren des oberen Austragsstroms aus der Trennkolonne für flüchtige organische Verbindungen zu dem Aldolreinigungsprozess.

3. Verfahren zur Behandlung von Abwasser von Neopentylglykol nach Anspruch 1, wobei der Aldolreinigungsprozess ein Prozess des Zirkulierens des getrennten nicht umgesetzten Isobutylaldehyds und des Katalysators zu dem Aldolreaktionsprozess ist.

4. Verfahren zur Behandlung von Abwasser von Neopentylglykol nach Anspruch 1, ferner umfassend einen Verseifungsreaktionsprozess des Verseifens des in dem Aldolextraktionsprozess erhaltenen Raffinats, um das Katalysatorsalz zu dem Katalysator zu reduzieren.

5. Verfahren zur Behandlung von Abwasser von Neopentylglykol nach Anspruch 4, ferner umfassend einen Katalysatorrückgewinnungsprozess des Destillierens des in dem Verseifungsreaktionsprozess reduzierten Katalysators, um Abwasser und den Katalysator zu trennen, und des Zirkulierens des getrennten Katalysators zu dem Aldolreinigungsprozess.

6. Verfahren zur Behandlung von Abwasser von Neopentylglykol nach Anspruch 5, ferner umfassend das Zuführen des aus dem Katalysatorrückgewinnungsprozess getrennten Abwassers zu der Trennkolonne für flüchtige organische Verbindungen.

7. Verfahren zur Behandlung von Abwasser von Neopentylglykol nach Anspruch 1, wobei der Extraktionsmittelrückgewinnungsprozess ein Prozess des Zuführens des Katalysators und des aus dem Neopentylglykolreinigungsprozess getrennten Extraktionsmittels zu der Katalysatorrückgewinnungskolonne, des Trennens des Abwassers, das den Katalysator, den Katalysator und das Extraktionsmittel enthält, des Einführens des Katalysators in den Aldolreinigungsprozess und des Einführens des Extraktionsmittels in den Aldolextraktionsprozess ist.

8. Verfahren zur Behandlung von Abwasser von Neopentylglykol nach Anspruch 1,
wobei der Neopentylglykolreinigungsprozess ein Prozess des weiteren Trennens von Hydroxypivalinsäure-Neopentylglykolester (HPNE) aus dem zweiten Reaktionsprodukt ist, und
ein Hydroxypivalinsäure-Neopentylglykolester-Reinigungsprozess des Destillierens eines Stroms, der Hydroxypivalinsäure-Neopentylglykolester (HPNE) enthält, der aus dem Neopentylglykolreinigungsprozess getrennt wurde, und des Zirkulierens einer kleinen Menge von Neopentylglykol zu dem Neopentylglykolreinigungsprozess, um Hydroxypivalinsäure-Neopentylglykolester zu erhalten, ferner enthalten ist.

9. Verfahren zur Behandlung von Abwasser von Neopentylglykol nach Anspruch 1, wobei der Katalysator Triethylamin (TEA) enthält.

10. Verfahren zur Behandlung von Abwasser von Neopentylglykol nach Anspruch 1, wobei das Extraktionsmittel 2-Ethylhexanol (2-EH) enthält.

11. Verfahren zur Behandlung von Abwasser von Neopentylglykol nach Anspruch 1, wobei eine Menge des Katalysators in dem unteren Austragsstrom aus der Trennkolonne für flüchtige organische Verbindungen 5,0 Gew.-% oder weniger beträgt.

12. Verfahren zur Behandlung von Abwasser von Neopentylglykol nach Anspruch 1, ferner umfassend das Zuführen des unteren Austragsstroms aus der Trennkolonne für flüchtige organische Verbindungen zu einem Verdampfer, das Entfernen von Schlamm und das Zuführen einer oberen Fraktion des Verdampfers, aus dem der Schlamm entfernt wurde, zu dem Abwasserbehandlungssystem.

## Revendications

1. Procédé de traitement des eaux usées de néopentylglycol, le procédé comprenant:
un processus de réaction aldolique consistant à effectuer une réaction de condensation aldolique entre une solution aqueuse de formaldéhyde et de l'isobutylaldéhyde en présence d'un catalyseur afin d'obtenir un premier produit de réaction comprenant de l'hydroxypivaldéhyde;
un processus d'extraction aldolique consistant à mettre le premier produit de réaction en contact avec un agent d'extraction afin d'obtenir un raffinat comprenant un sel catalytique et un extrait comprenant de l'hydroxypivaldéhyde;
un processus de purification aldolique consistant à distiller l'extrait pour le séparer en eaux usées contenant le catalyseur, l'isobutylaldéhyde n'ayant pas réagi et le catalyseur, et l'hydroxypivaldéhyde;
un procédé de réaction d'hydrogénation consistant à hydrogéner l'hydroxypivaldéhyde séparé dans le procédé de purification aldolique pour obtenir un deuxième produit de réaction comprenant du néopentylglycol; et
un procédé de purification du néopentylglycol consistant à distiller le deuxième produit de réaction à séparer en l'agent d'extraction et le catalyseur, les eaux usées contenant le catalyseur et le néopentylglycol, et à acheminer l'agent d'extraction et le catalyseur séparés vers une colonne de récupération de l'agent d'extraction dans laquelle un procédé de récupération de l'agent d'extraction est effectué pour séparer les eaux usées contenant le catalyseur et obtenir le néopentylglycol séparé, et comprenant:
récupérer les eaux usées contenant le catalyseur à partir d'un ou plusieurs processus du processus de purification aldolique, du processus de purification du néopentylglycol et du processus de récupération de l'agent d'extraction, et fournir les eaux usées comprenant le catalyseur à une colonne de séparation des composés organiques volatils;
la distillation des eaux usées contenant le catalyseur dans la colonne de séparation des composés organiques volatils afin d'obtenir un flux de décharge supérieur contenant le catalyseur et un flux de décharge inférieur contenant les eaux usées dont le catalyseur a été éliminé; et
fournir le flux de décharge inférieur provenant de la colonne de séparation des composés organiques volatils à un système de traitement des eaux usées afin de traiter les eaux usées.

2. Procédé de traitement des eaux usées de néopentylglycol selon la revendication 1, comprenant en outre: la circulation du flux de décharge supérieur provenant de la colonne de séparation des composés organiques volatils vers le processus de purification aldolique.

3. Procédé de traitement des eaux usées de néopentylglycol selon la revendication 1, dans lequel le processus de purification aldolique est un processus consistant à faire circuler l'isobutylaldéhyde non réagi séparé et le catalyseur vers le processus de réaction aldolique.

4. Procédé de traitement des eaux usées contenant du néopentylglycol selon la revendication 1, comprenant en outre un processus de réaction de saponification consistant à saponifier le raffinat obtenu dans le processus d'extraction aldolique afin de réduire le sel catalytique au catalyseur.

5. Procédé de traitement des eaux usées de néopentylglycol selon la revendication 4, comprenant en outre un processus de récupération du catalyseur consistant à distiller le catalyseur réduit dans le processus de réaction de saponification afin de séparer les eaux usées et le catalyseur, et à faire circuler le catalyseur séparé vers le processus de purification aldolique.

6. Procédé de traitement des eaux usées de néopentylglycol selon la revendication 5, comprenant en outre l'étape consistant à fournir les eaux usées séparées lors du processus de récupération du catalyseur à la colonne de séparation des composés organiques volatils.

7. Procédé de traitement des eaux usées de néopentylglycol selon la revendication 1, dans lequel le processus de récupération de l'extractant est un processus consistant à fournir le catalyseur et l'extractant séparés du processus de purification du néopentylglycol à la colonne de récupération du catalyseur, à séparer les eaux usées comprenant le catalyseur, le catalyseur et l'extractant, à introduire le catalyseur dans le processus de purification aldolique et à introduire l'extractant dans le processus d'extraction aldolique.

8. Procédé de traitement des eaux usées de néopentylglycol selon la revendication 1,
dans lequel le processus de purification du néopentylglycol est un processus consistant à séparer davantage l'ester d'hydroxypivalate de néopentylglycol (HPNE) du deuxième produit de réaction, et
un processus de purification de l'ester d'hydroxypivalate de néopentylglycol consistant à distiller un flux comprenant l'ester d'hydroxypivalate de néopentylglycol (HPNE) séparé du processus de purification du néopentylglycol et à faire circuler une petite quantité de néopentylglycol vers le processus de purification du néopentylglycol afin d'obtenir l'ester d'hydroxypivalate de néopentylglycol est en outre inclus.

9. Procédé de traitement des eaux usées de néopentylglycol selon la revendication 1, dans lequel le catalyseur comprend de la triéthylamine (TEA).

10. Procédé de traitement des eaux usées contenant du néopentylglycol selon la revendication 1, dans lequel l'agent d'extraction comprend du 2-éthylhexanol (2-EH).

11. Procédé de traitement des eaux usées contenant du néopentylglycol selon la revendication 1, dans lequel la quantité de catalyseur dans le flux de décharge inférieur provenant de la colonne de séparation des composés organiques volatils est inférieure ou égale à 5,0 % en poids.

12. Procédé de traitement des eaux usées contenant du néopentylglycol selon la revendication 1, comprenant en outre l'étape consistant à fournir le flux de décharge inférieur provenant de la colonne de séparation des composés organiques volatils vers un évaporateur, à éliminer les boues et à fournir une fraction supérieure de l'évaporateur dont les boues ont été éliminées vers le système de traitement des eaux usées.
